(19) Europäisches Patentamt
European Patent Office
Office européen des brevets

(11) Publication number : **0 384 594 B1**

(12) # EUROPEAN PATENT SPECIFICATION

(45) Date of publication of patent specification : **27.10.93 Bulletin 93/43**

(51) Int. Cl.$^5$ : **C07C 275/64,** A61K 31/17

(21) Application number : **90301090.8**

(22) Date of filing : **02.02.90**

(54) **Anti-inflammatory aryl derivatives.**

The file contains technical information submitted after the application was filed and not included in this specification

(30) Priority : **03.02.89 GB 8902472**
**25.08.89 GB 8919314**
**03.11.89 GB 8924867**

(43) Date of publication of application : **29.08.90 Bulletin 90/35**

(45) Publication of the grant of the patent : **27.10.93 Bulletin 93/43**

(84) Designated Contracting States : **AT BE CH DE DK ES FR GB GR IT LI LU NL SE**

(56) References cited : **EP-A- 0 196 184**

(56) References cited :
**EP-A- 0 279 281**
**EP-A- 0 292 699**
**EP-A- 0 299 761**
**FR-A- 2 599 739**

(73) Proprietor : **THE WELLCOME FOUNDATION LIMITED**
**Unicorn House 160 Euston Road**
**London NW1 2BP (GB)**

(72) Inventor : **Jackson, William Paul**
**Wellcome Foundation Limited Langley Court**
**Beckenham, Kent (GB)**
Inventor : **Salmon, John Anthony**
**Wellcome Foundation Limited Langley Court**
**Beckenham, Kent (GB)**

(74) Representative : **Garrett, Michael et al**
**The Wellcome Foundation Limited Group Patents and Agreements Langley Court**
**Beckenham Kent BR3 3BS (GB)**

EP 0 384 594 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel aryl hydroxyurea compounds having anti-inflammatory activity, to processes for their preparation, to pharmaceutical formulations containing them and, optionally, one or more other pharmacologically active ingredients and to the use of these compounds in medicine.

A class of compounds defined in European Patent Specification 0055418 are described therein as dual inhibitors of the 5-lipoxygenase and cyclo-oxygenase enzymes of the mammalian arachidonic acid metabolic pathway and were found to exhibit anti-inflammatory and related activities. Other compounds which have been described as 5-lipoxygenase and/or cyclo-oxygenase inhibitors include certain naphthyloxy derivatives, for example, as described in U.S. Patent 3,740,437 or in Proc. Ann. Symp. Inst. Basic Med. Sci., Royal College of Surgeons of England, October 1982, pp. 263-274. Compounds described in the latter reference include the compound known as nafazatrom.

European Patent Specification 0196184 describes a class of hydroxamic acid derivatives which are inhibitors of the 5-lipoxygenase enzyme. European Patent Specification 0299761 describes a sub-class of α-alkylated N-alkanoylhydroxamic acids falling within the scope of the class of hydroxamic acid derivatives described in European Patent Specification 0196184, the compounds of said sub-class having particularly desirable pharmacological properties with regard to their ability to inhibit 5-lipoxygenase.

We have now found within the class of compounds described in European Patent Specification 0196184 a further sub-class of compounds, characterised as α-methylated N-hydroxyureas, having particularly advantageous pharmacological and physical properties, for example, with respect to their 5-lipoxygenase inhibitory and anti-oxidant properties and the long duration of action demonstrated by both of their enantiomers.

According to the present invention, therefore, there is provided a compound of formula (I)

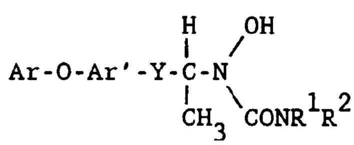

(I)

wherein
Ar is 4-halophenyl;
Ar' is 1,3- or 1,4-phenylene;
Y is (E)-CH=CH-; and
$R^1$ and $R^2$ are independently selected from hydrogen and $C_{1-4}$ alkyl;
and base salts and physiologically functional derivatives thereof.

It will be appreciated that compounds of formula (I) and their base salts may exist in (R) or (S) enantiomeric forms. The present invention therefore includes within its scope each of the individual (R) and (S) enantiomers of the compounds of formula (I) and their base salts substantially free, ie associated with less than 5%, of the other enantiomer and mixtures of such enantiomers in any proportions including racemic mixtures containing substantially equal amounts of the two enantiomers.

Preferred compounds of the invention include those wherein
Ar is 4-fluorophenyl; and/or
Ar' is 1,3-phenylene; and/or
$R^1$ and $R^2$ are both hydrogen;
and base salts and physiologically functional derivatives thereof.

A particularly preferred compound of formula (I) having exceptionally advantageous 5-lipoxygenase inhibitory and anti-oxidant properties, particularly in respect of its duration of action, is (E)-N-{1-methyl-3-[3-(4-fluorophenoxy)phenyl]prop-2-en-1-yl}-N-hydroxyurea, preferably in the form of a racemic mixture of the two enantiomers.

Base salts of compounds of formula (I) which are suitable for use in medicine are those wherein the cation is physiologically acceptable. However, base salts having non-physiologically acceptable cations are within the ambit of the present invention, either for use in non-medical applications or as intermediates in the preparation of compounds of formula (I) and their physiologically acceptable salts and physiologically functional derivatives.

Salts according to the invention include ammonium salts, alkali metal salts such as those of sodium and potassium, alkaline earth metal salts such as those of calcium and magnesium, salts with organic bases such as dicyclohexylamine and N-methyl-D-glucamine, and salts with amino acids, such as arginine and lysine.

The above compounds of formula (I) and their physiologically acceptable base salts and physiologically functional derivatives are hereinafter referred to as "compounds of the invention" in relation to the therapeutic and pharmaceutical aspects of the invention discussed below.

According to further aspects of the invention, there are provided:

(a) compounds of the invention for use in therapy;

(b) pharmaceutical formulations containing a compound of the invention, at least one pharmaceutical carrier or excipient and, optionally, one or more other therapeutic ingredients;

(c) the use of a compound of the invention in the manufacture of a medicament for the prophylaxis or treatment of a clinical condition for which a 5-lipoxygenase inhibitor or anti-oxidant is indicated;

(d) the use of a compound of the invention in the manufacture of a medicament for the prophylaxis or treatment of

(i) a spasmogenic condition,

(ii) an allergic condition,

(iii) tumour formation,

(iv) a condition involving blood platelet aggregation,

(v) an inflammatory condition, or

(vi) atherosclerosis;

(e) processes for the preparation of the compounds of the invention.

By virtue of their 5-lipoxygenase inhibitory properties, the compounds of the invention find application in the prophylaxis or treatment of clinical conditions for which an inhibitor of the lipoxygenase-mediated arachidonic acid metabolic pathway is indicated, especially

(i) spasmogenic conditions,

(ii) allergic conditions,

(iii) tumour formation,

(iv) conditions involving blood platelet aggregation, and

(v) inflammatory conditions.

These will be discussed in turn.

(i) Examples of spasmogenic conditions are those involving smooth muscle tissue, especially airway smooth muscle constriction such as intrinsic asthma (including idiopathic bronchial asthma and cardiac asthma), bronchitis and arterial smooth muscle constriction such as coronary spasm (including that associated with myocardial infarction, which may or may not lead to left ventricular failure resulting in cardiac asthma) and cerebral spasm or 'stroke'. Other examples include bowel disease caused by abnormal colonic muscular contraction such as the conditions known as 'irritable bowel syndrome', 'spastic colon' and 'mucous colitis'.

(ii) Examples of allergic conditions are extrinsic asthma, allergic skin diseases having a total or partial allergic origin, such as eczema, allergic bowel diseases (including coeliac disease), allergic eye conditions, such as hayfever (which may additionally or alternatively affect the upper respiratory tract), and allergic conjunctivitis.

(iii)Examples of tumours are skin neoplasms, mastocytoma and other forms of cellular proliferation, both benign and malignant. It is to be noted that the effectiveness of the present compounds in the prophylaxis and treatment of tumours may arise from properties in addition to 5-lipoxygenase inhibition which also inhibit cell proliferation.

(iv) Examples of conditions involving blood platelet aggregation are those resulting from thrombosis, including 'strokes' having a total or partial thrombotic origin, coronary thrombosis, phlebitis and phlebothrombosis (the latter two conditions also possibly being associated with inflammation).

(v) Examples of inflammatory conditions are those of the (a) lungs, (b) joints, (c) eyes, (d) bowel, (e) skin and (f) heart, particularly those associated with the infiltration of leucocytes into inflamed tissue. Examples of such inflammatory conditions are:

(a) Inflammatory lung conditions include asthma, bronchitis and cystic fibrosis (which may additionally or alternatively involve the bowel or other tissue(s)).

(b) Inflammatory joint conditions include rheumatoid arthritis, rheumatoid spondylitis, osteoarthritis, gouty arthritis and other arthritic conditions.

(c) Inflammatory eye conditions include uveitis (including iritis) and conjunctivitis.

(d) Inflammatory bowel conditions include Crohn's disease, ulcerative colitis and distal proctitis.

(e) Inflammatory skin diseases include those associated with cell proliferation, such as psoriasis, eczema and dermatitis (whether or not of allergic origin).

(f) Inflammatory conditions of the heart include coronary infarct damage.

Other inflammatory conditions include tissue necrosis in chronic inflammation and tissue rejection following transplant surgery.

By virtue of their anti-oxidant properties, the compounds of the invention also find application in the prophylaxis or treatment of clinical conditions for which a cytoprotective agent having such properties is indicated, for example, atherosclerosis and arthritis.

In view of their unique properties the compounds of the invention may also be employed in the prophylaxis or treatment of bacterial and fungal infections, dysmenorrhoea, multiple sclerosis and clinical conditions for which an immunosuppressant, anti-convulsant, or analgesic is indicated. The compounds of the invention also exhibit hypocholesterolaemic activity.

The amount required of the compound of the invention (hereinafter referred to as the active ingredient) to achieve a therapeutic effect will, of course, vary with the particular compound, the route of administration, the subject under treatment, and the particular disorder or disease being treated. A suitable dose for a mammal suffering from, or likely to suffer from, any of the clinical conditions described hereinbefore is in the range 0.1μg to 500mg of compound/kilogram bodyweight. In the case of systemic administration, the dose is typically in the range 0.5 to 500mg of compound/kilogram bodyweight, the most preferred dosage being 0.5 to 50mg/kg bodyweight, for example, 5 to 25mg/kg, administered two or three times daily. In the case of topical administration, e.g. to the skin or eye, a suitable dose is in the range 0.1ng-100μg of base per kilogram, typically about 0.1 μg/kg.

In the case of oral dosing for the prophylaxis or treatment of airway smooth muscle constriction, for example, in asthma or bronchitis, a suitable dose of the compound of the invention may be as specified in the preceding paragraph, but most preferably is from 1mg to 10mg of base per kilogram bodyweight, the most preferred dosage being from 1mg to 5mg/kg bodyweight, for example, from 1 to 2 mg/kg. In the case of pulmonary administration, the dose is typically in the range 2μg to 100mg/kg, for example, from 20μg to 0.5mg/kg, especially from 0.1 to 0.5 mg/kg.

While it is possible for the active ingredient to be administered alone, it is preferable to present it as a pharmaceutical formulation comprising a compound of the invention in association with a pharmaceutically acceptable carrier or excipient and, optionally, one or more other therapeutic ingredients. The carrier or excipient must, of course, be compatible with the other ingredients in the formulation and must not be detrimental to the recipient. The active ingredient may comprise from 0.1% to 99.9% by weight of the formulation. Typical unit doses of a formulation according to the invention contain from 0.1mg to 1g of the active ingredient. For topical administration, the active ingredient preferably constitutes from 1% to 2% by weight of the formulation, but the active ingredient may constitute as much as 10% w/w. Formulations suitable for nasal or buccal administration, typically contain from 0.1 to 20% w/w, for example, 2% w/w of the active ingredient.

Formulations according to the invention include those in a form suitable for oral, pulmonary, ophthalmic, rectal, parenteral (including subcutaneous, intramuscular and intravenous), intra-articular, topical, or nasal/buccal administration.

The formulations of the invention may conveniently be presented in unit dosage form and may be prepared by any method well known in the art of pharmacy. All such methods include the step of bringing the active ingredient into association with a carrier which constitutes one or more accessory ingredients. In general, the formulations are prepared by uniformly and intimately bringing the active ingredient into association with a liquid carrier or a finely divided solid carrier, or both, and then, if desired, shaping the product into the required form.

Formulations according to the present invention which are suitable for oral administration may be in the form of discrete units such as capsules, cachets, tablets, or lozenges, each containing a predetermined amount of the active ingredient; in the form of a powder or granules; in the form of a solution or a suspension in an aqueous or non-aqueous liquid; or in the form of an oil-in-water or water-in-oil emulsion. The active ingredient may also be in the form of a bolus, electuary, or paste.

A tablet may be made by compressing or moulding the active ingredient, optionally with one or more accessory ingredients. Compressed tablets may be prepared by compressing, in a suitable machine, the active ingredient in a free-flowing form such as a powder or granules, optionally mixed with a binder, lubricant, inert diluent, and/or surface active or dispersing agent. Moulded tablets may be made by moulding, in a suitable machine, a mixture of the powdered active ingredient and a suitable carrier moistened with an inert liquid diluent.

Formulations for rectal administration may be in the form of a suppository incorporating the active ingredient and a carrier such as cocoa butter, or in the form of an enema.

Formulations suitable for parenteral administration typically comprise a sterile aqueous preparation of the active ingredient which is preferably isotonic with the blood of the recipient.

Formulations suitable for intra-articular administration may be in the form of a sterile aqueous preparation of the active ingredient, which latter may be in microcrystalline form, for example, an aqueous microcrystalline

suspension. Liposomal formulations and biodegradable polymer systems may also be used to present the active ingredient for both intra-articular and ophthalmic administration.

Formulations suitable for topical administration include liquid and semi-liquid preparations such as liniments, lotions and applications; oil-in-water and water-in-oil emulsions such as creams, ointments and pastes; and solutions and suspensions such as drops. For example, for ophthalmic administration, the active ingredient may be presented as aqueous eye drops, for example, in the form of a 0.1 - 1.0% w/v solution.

Suitable formulations for administration by inhalation include fine particle dusts or mists which may be generated by means of various types of metered dose pressurised aerosols, nebulisers, or insufflators.

For pulmonary administration via the mouth, the particle size of the powder or droplets is typically in the range 0.5 - 10μm, preferably 1 - 5μm, to ensure delivery into the bronchial tree. For nasal administration, a particle size in the range 10 - 500μM is preferred to ensure retention in the nasal cavity.

Metered dose inhalers are pressurised aerosol dispensers, typically containing a suspension or solution formulation of the active ingredient in a liquefied propellant. During use these devices dishcarge the formulation through a valve adapted to deliver a metered volume, typically from 10 to 150μl, to produce a fine particle spray containing the active ingredient. Suitable propellants include certain chlorofluorocarbon compounds, for example, dichlorodifluoromethane, trichlorofluoromethane, dichlorotetrafluoroethane and mixtures thereof. The formulation may additionally contain one or more co-solvents, for example, ethanol, surfactants, such as oleic acid or sorbitan trioleate, antioxidants and suitable flavouring agents.

Nebulisers are commercially available devices that transform solutions or suspensions of the active ingredient into a therapeutic aerosol mist either by means of acceleration of a compressed gas through a narrow venturi orifice, typically air or oxygen, or by means of ultrasonic agitation. Suitable formulations for use in nebulisers consist of the active ingredient in a liquid carrier and comprising up to 40% w/w of the formulation, preferably less than 20% w/w. The carrier is typically water or a dilute aqueous alcoholic solution, preferably made isotonic with body fluids by the addition of, for example, sodium chloride. Optional additives include preservatives if the formulation is not prepared sterile, for example, methyl hydroxybenzoate, antioxidants, flavouring agents, volatile oils, buffering agents and surfactants.

Suitable formulations for administration by insufflation include finely comminuted powders which may be delivered by means of an insufflator or taken into the nasal cavity in the manner of a snuff. In the insufflator, the powder is contained in capsules or cartridges, typically made of gelatin or plastic, which are either pierced or opened in situ and the powder delivered by air drawn through the device upon inhalation or by means of a manually-operated pump. The powder employed in the insufflator consists either solely of the active ingredient or of a powder blend comprising the active ingredient, a suitable powder diluent, such as lactose, and an optional surfactant. The active ingredient typically comprises from 0.1 to 100 w/w of the formulation.

In addition to the aforementioned ingredients, formulations according to the invention may include one or more additional ingredients such as diluents, buffers, flavouring agents, binders, surface active agents, thickeners, lubricants, preservatives, for example, methyl hydroxybenzoate, anti-oxidants and emulsifying agents.

The compounds of the invention may advantageously be employed in combination with one or more other therapeutic ingredients selected from an antibiotic (for example, an anti-bacterial), anti-fungal, or anti-viral agent, an anti-histamine (particularly a peripherally-acting anti-histamine), or a non-steroidal anti-inflammatory drug (NSAID).

The compounds of these combinations may be administered simultaneously, for example, in the same formulation or in separate formulations, or sequentially within a sufficiently short time interval to achieve the desired combined therapeutic effect. When the compounds are employed in the same formulation, a formulation according to the invention may be employed containing, in addition to a compound of the invention, the further ingredient(s).

The combination with an anti-histamine is particularly favoured for use in the prophylaxis and treatment of asthma. A suitable anti-histamine may be selected from the compounds described in European Patent Applications Nos.0085959 and 0117302. The amount and dosage regime for such an anti-histamine may be chosen from any of those recited in these two documents. Especially preferred are the anti-histamines (E)-3-(6-(3-pyrrolidino-1-(4-tolyl)prop-1E-enyl. (2-pyridyl)acrylic acid and (E)-3-(6-(3-pyrrolidino-1-(4-tolyl)prop-1E-enyl-(2-pyridyl)propionic acid. Another preferred anti-histamine is (E)-1-(4-methylphenyl)-1-(2-pyridyl)-3-pyrrolidino-prop-1-ene, otherwise known as triprolidine.

The combination with an NSAID is particularly favoured for use in the prophylaxis and treatment of inflammatory joint conditions such as those referred to above. It has been found that the use of such combinations in the treatment of inflammatory joint conditions produces a therapeutic effect superior to that obtained using either drug alone. In particular, the reduction in joint swelling and the decrease in the thickness of the synovial lining of the joint are significantly improved when using the combination therapy. Furthermore, there is a reduction in the loss of proteoglycan from the articular cartilage of the joint and reduced infiltration of polymorphonuclear and other leucocytes into the synovial tissue which effects are not observed when using either drug alone.

NSAID's, particularly at high dose levels, are known to cause gastric ulceration. In addition to the improved therapeutic effect observed using an NSAID in combination with a compound of the invention, the anti-oxidant properties of the latter provide cytoprotection against the ulcerative effect of the NSAID so that larger doses of the NSAID may be administered with less risk of causing ulceration.

Thus the present invention further provides a pharmaceutical formulation suitable for oral administration or intra-articular injection comprising a compound of the invention (as hereinbefore defined) and at least one NSAID. Suitable NSAID's include acemetacin, alminoprofen, amfenac sodium, aminoprofen, antitrazafen, antrafenine, auranofin, bendazac lysinate, benzydamine, beprozin, broperamole, bufezolac, carprofen, cinmetacin, ciproquazone, clidanac, cloximate, dazidamine, deboxamet, delmetacin, detomidine, dexindoprofen, diacerein, diclofenac, difisalamine, difenpyramide, emorfozone, enfenamic acid, enolicam, epirizole, etersalate, etodolac, etofenamate, fanetizole mesylate, fenclofenac, fenclorac, fendosal, fenifumizole, fentiazac, feprazone, floctafenine, flunixin, flunoxaprofen, fluproquazone, flurbiprofen, fopirtoline, fosfosal, furcloprofen, furofenac, glucametacin, guaimesal, ibuprofen, ibuproxam, indomethacin, isofezolac, isonixim, isoprofen, isoxepac, isoxicam, ketoprofen, lefetamine HCl, leflunomide, lofemizole, lonazolac calcium, lotifazole, loxoprofen, lysin clonixinate, meclofenamate sodium, meseclazone, miroprofen, nabumetone, naproxen, nictindole, nimesulide, orpanoxin, oxametacin, oxapadol, oxaprozin, perisoxal citrate, pimeprofen, pimetacin, piproxen, pirazolac, pirfenidone, piroxicam, pirprofen, pranoprofen, proglumetacin maleate, proquazone, pyridoxiprofen, sudoxicam, suprofen, talmetacin, talniflumate, tenoxicam, thiazolinobutazone, thielavin B, tiaprofenic acid, tiaramide HCl, tiflamizole, timegadine, tioxaprofen, tolfenamic acid, tolpadol, tryptamid, ufenamate and zidometacin.

Preferred such NSAID's are naproxen, flurbiprofen, ketoprofen, aminoprofen, carprofen, clidanac, dexindoprofen, diclofenac, enfenamic acid, fenclofenac, flunoxaprofen, ibuprofen, indomethacin, isoprofen, loxoprofen, meclofenamate sodium, miroprofen, piroxicam, pirprofen, pranoprofen, suprofen, tiaprofenic acid, tioxaprofen and zidometacin.

Particularly preferred NSAID's for use according to this aspect of the invention are clidanac, diclofenac, ibuprofen, indomethacin, suprofen, naproxen, piroxicam, flurbiprofen, ketoprofen and tiaprofenic acid.

The following NSAID's, designated by company code number (Chemical Abstracts Index Guide, 1989), may also be used: 480156S, AA861, AD1491, AD1590, AFP802, AFP860, AHR6293, A177B, AP504, AU8001, BAYo8276, BPPC, BW540C, BW755C, CHINOIN 127, CN100, CO893XX, CPP, D10242, DKA9, DV17, EB382, EGYT2829, EL508, F1044, FZ, GP53633, GP650, GV3658, HG/3, ITC1, ITF, ITF182, KB1043, KC8973, KCNTE16090, KME4, LA2851, LT696, LU20884, M7074, MED15, MG18311, MR714, MR897, MY309, NO164, ONO3144, PR823, PV102, PV108, QZ16, R830, RS2131, RU16029, RU26559, RUB265, SCR152, SH440, SIR133, SIR136, SIR92, SPAS510, SQ27239, ST281, SX1032, SY6001, SaH46798, TA60, TAI901, TEI615, TVX2706, TVX960, TZI615, U60257, UR2310, WY23205, WY41770, YM09561, YM13162, YS1033 and ZK31945.

Further NSAID's include the salicylates, specifically aspirin, and the phenylbutazones and pharmaceutically acceptable salts thereof.

Typically a formulation according to this aspect of the invention comprises a non-toxic cytoprotective amount of a compound of the invention and an effective non-toxic (but potentially ulcerogenic in the absence of the compound of the invention) amount of the NSAID.

The quantity of NSAID in the formulation will depend on the intended effect thereof and will be selected by the physician as appropriate to the age, body weight, general health, etc. of the patient. The quantity of the compound of the invention to be included in the composition is selected to be compatible with the amount of NSAID and to provide sufficient cytoprotective effect to ameliorate or prevent the gastric ulcerogenic effect of the NSAID. Typical amounts of compounds of the invention for use in conjunction with an NSAID in oral or intra-articular formulations are as indicated earlier.

According to a further aspect of the invention, there is provided a process for preparing the compound of the invention which comprises reacting a compound of formula (II)

$$R^3NHOH \qquad (II)$$

wherein $R^3$ is a group of formula

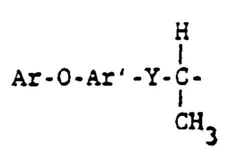

as defined for a compound of formula (I), with a suitable agent or agents and under such reactiop conditions as to effect conversion of the N-hydrogen to an N-CONR$^1$R$^2$ group;
and optionally converting the compound of the invention so formed to a corresponding salt thereof.

The reaction is typically carried out by treatment of the compound of formula (II) with a Group I cyanate, for example, potassium cyanate, in a non-polar solvent, such as tetrahydrofuran (THF), in the presence of acid.

Compounds of formula (II) may be prepared by oximation of the corresponding ketone using, for example, hydroxylamine in a suitable polar solvent, such a methanol, followed by reduction of the oxime in situ using, for example, sodium cyanoborohydride/oxalic acid. The appropriate ketone may be obtained commercially or by reaction of the corresponding aldehyde with, for example, aqueous sodium hydroxide (NaOH)/acetone or dimethyl acetylmethylphosphonate/anhydrous potassium carbonate in a suitable solvent such as THF. The appropriate aldehyde may be obtained commercially or, for example, by lithiation of the corresponding bromo compound followed by treatment with dimethylformamide (DMF). The bromo compound may be obtained commercially or, for example, by reacting a compound of formula ArOS, where Ar is as hereinbefore defined and S is a Group I metal, with a compound of formula Ar'Br, where Ar' is as hereinbefore defined, in a suitable solvent such as 1-methyl-2-pyrrolidinone. Lithiation is typically carried out using butyl lithium at low temperature for example, -60°C, in a non-polar solvent such as THF. The aldehyde may also be obtained by treating the corresponding dibromomethyl compound with ethylenediaminetetraacetic acid disodium salt (EDTA.2Na)/calcium carbonate in a polar solvent system such as ethanol/water. The dibromomethyl compound may be obtained commercially or, for example, by irradiating a compound of formula ArOAr'$CH_3$, where Ar, and Ar' are as hereinbefore defined, in a non-polar solvent, such as $CCl_4$, in the presence of N-bromosuccinimide (NBS)/azoisobutyronitrile (AIBN).

The individual enantiomers of the compound of the invention may be obtained by separation of the components of the racemic mixture, for example, by means of a chiral chromatography column or by preparing and separating suitable diastereoisomers, or by direct synthesis from the corresponding chiral compound of formula (II) by the method described above.

Chiral compounds of formula (II) may conveniently be obtained by acid or base hydrolysis of the corresponding chiral bis-blocked compound, for example, the -N($CO_2$Me)O$CO_2$Me compound or -N(BOC)OBOC compound where BOC is t-butoxycarbonyl. The latter compound may be obtained by treating the appropriate chiral alcohol A with HN(BOC)O3OC in the presence of triphenylphosphine/diethyl azodicarboxylate (DEAD) at low temperature in a non-polar solvent, such as toluene, or by reacting the corresponding chiral compound of formula (III)

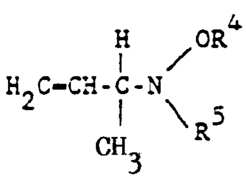

(III)

wherein R$^4$ and R$^5$ are both t-butoxycarbonyl groups, with a compound of formula (IV)

Ar-O-Ar'-T (IV)

wherein Ar, and Ar' are as defined for the compound of the invention and T is a suitable leaving group, such as bromo, typically at elevated temperature in the presence of palladium (II) acetate, tri(o- tolyl)phosphine and a suitable base, for example, tri(n-butyl) amine.

Chiral compounds of formula (III) may conveniently be obtained by treatment of the appropriate chiral alcohol B with HN(BOC)OBOC in the presence of triphenylphosphine/DEAD or by partial reduction of the corresponding chiral alkyne by, for example, quantitative hydrogenation over a suitable catalyst, such as palladium on barium sulphate, in a basic solvent such as pyridine. The chiral alkyne may be obtained by treatment of the corresponding chiral alcohol C with HN(BOC)OBOC in the presence of triphenylphosphine/DEAD. The appropriate chiral alcohols A, B and C may be obtained commercially or prepared from the corresponding ketones by means of suitable chiral reducing agents (JACS 109, 7925 (1987)) Alternatively, they may be obtained from the racemic alcohols by derivatisation and selective hydrolysis (JCS Chem. Comm. 597 (1988)) or by the separation of suitable diastereoisomers (J. Med. Chem. 31, 1558 (1988)). Compounds of formula (IV) are available commercially or may be prepared by methods described in the literature.

Optional conversion of the compound of the invention to a corresponding base salt may conveniently be effected by reaction with the appropriate base.

For a better understanding of the invention, the following Examples are given by way of illustration.

SYNTHETIC EXAMPLES

Synthetic Example 1

Preparation of (E)-N-{3-[3-(4-fluorophenoxy)phenyl]-1(R,S)-methyl prop-2-en-1-yl}-N-hydroxyurea

(a) 3-(4-Fluorophenoxy)toluene

A solution of 4-fluorophenol (112.0g, Aldrich) in anisole (250ml) was added dropwise over 1 hour to a stirred suspension of NaH (26.4g) in anisole (250ml). When addition was complete, the mixture was stirred at room temperature for 16 hours. 3-Bromotoluene (188.1g, Aldrich), copper(I) chloride (50.0g) and TDA-1 (tris[2-(2-methoxyethoxy)ethyl]amine, 158ml) were then added seqentially over 30 minutes. When addition was complete, the mixture was refluxed for 22 hours, then cooled to room temperature and ether (2000ml) added. The mixture was filtered through Hyflo and the filtrate washed with 2M aqueous HCl, 1M aqueous NaOH and water, then dried and evaporated in vacuo. The remaining oil was distilled to give the desired product (101.9g) at bp 129-134°C/9mm Hg.

(b) 1-Dibromomethyl-3-(4-fluorophenoxy)benzene

A solution of the product from step (a) (101.9g) and NBS (206.7g) in $CCl_4$ (500ml) was heated to reflux. AIBN (100mg) was then added and the mixture irradiated with a medium pressure lamp at 254nm whilst refluxing for 7 hours. Further portions of AIBN (100mg) were added after 0.5, 1, 2, 4 and 6 hours. The mixture was cooled to room temperature, filtered and the filtrate evaporated in vacuo to give the desired product as an orange oil (195.3g).

(c) 3-(4-Fluorophenoxy)benzaldehyde

A mixture of the product from step (b) (181.8g), $CaCO_3$ (151.7g) and EDTA.2Na. $2H_20$ (37.7g) in ethanol/water (1140ml/284ml) was refluxed for 20 hours. The mixture was cooled to room temperature, filtered and the filtrate evaporated in vacuo. The remaining oil was taken up in ether (500ml), washed with 2M aqueous HCl, saturated aqueous $NaHCO_3$ and water, dried and evaporated in vacuo to give a viscous orange oil which was chromatographed twice through silica gel using ether/40-60 pet. ether (1:9) to give the desired product (69.6g).

(d) 1-[3-(4-Fluorophenoxy)phenyl]but-1-en-3-one

A mixture of the product from step (c) (69.6g), dimethyl acetylmethylphosphonate (56.2g) and anhydrous $K_2CO_3$ (88.9g) in THF (500ml) was refluxed for 24 hours. The mixture was cooled to room temperature, filtered and the filtrate evaporated in vacuo. The remaining oil was taken up in ether (500ml), washed with water, saturated aqueous $NaHCO_3$ and water, dried and evaporated in vacuo to give the desired product (83.4g).

(e) N-{1-[3-(4-Fluorophenoxy)phenyl]but-2-en-3-yl}hydroxylamine

Pyridine (63.6g) was added dropwise over 1 hour to a stirred solution of the product from step (d) (82.5g) and hydroxylamine hydrochloride (33.6g) in methanol (1000ml). When addition was complete, the mixture was stirred for 1.5 hours, then cooled in ice/water and anhydrous oxalic acid added (289.8g). Further methanol (750ml) was added and sodium cyanoborohydride (101.2g) added to the stirred mixture over 3.5 hours. When addition was complete, the mixture was stirred for 2 hours at 0-5°C and then 20 hours at room temperature. The mixture was filtered, the filtrate evaporated in vacuo and the remaining oil taken up in ether (1000ml), washed with water and cooled in ice/water. The pH of the mixture was adjusted to about 9 by the addition of 10M aqueous NaOH and the organic phase removed. The aqueous phase was extracted with ether and the organic phase and extract combined, washed with water, dried and evaporated in vacuo to give the desired product as a tan oil (92.7g) which crystallised on standing.

(f) (E)-N-{3-[3-(4-Fluorophenoxy)phenyl]-1(R,S)-methylprop-2-en-1-yl}-N-hydroxyurea

2M aqueous HCl (645ml) was added at 0°C over 1.5 hours to a stirred solution of the product step (e) (88.0g) and potassium cyanate (79.2g) in THF (750ml). When addition was complete, the mixture was stirred

at 0°C for 2 hours, then allowed to separate. The organic phase was removed, the aqueous phase was extracted with ether and the organic phase and extracts were combined, washed with saturated aqueous $NaHCO_3$ and water, dried and evaporated in vacuo to give a colourless sticky solid. The latter was triturated with ether/40-60 pet. ether (3:2), then recrystallised from ethyl acetate/40-60 pet. ether (2:1) to give a colourless solid (67.8g) of mp 133.5-135 °C (dec.). 200MHz $^1H$ NMR and IR were consistent with the desired product. Analysis: C 64.81% (64.54%); H 5.43% (5.42%); N 8.52% (8.86%).

Synthetic Example 2

Preparation of (E)-N-{3-[3-(4-fluorophenoxy)phenyl]-1(R)-methylprop-2-en-1-yl}-N-hydroxyurea

(a) But-3-yn-2(S)-ol

But-3-yn-2(S)-ol was obtained by the resolution of (±)-but-3-yn-2-ol (Aldrich) according to the procedure described in J. Med. Chem. 31, 1558 (1988).

(b) N,O-Bis(t-butoxycarbonyl)-N-[but-3-yn-2(R)-yl]hydroxylamine

DEAD (180.0g) was added dropwise at -78°C over 1 hour to a stirred solution of but-3-yn-2(S)-ol from step (a) (55.0g), triphenylphosphine (258.0g) and N,O-bis(t-butoxycarboxyl)hydroxylamine (183.0g, Fluka) in toluene (1500ml). When addition was complete, the mixture was allowed to warm to room temperature and then stirred for 2 hours. 40-60 Pet. ether (1500ml) was added, the mixture filtered through Hyflo and the filtrate evaporated in vacuo to give the desired product as an oil (200.0g) which crystallised on standing.

(c) N,O-Bis(t-butoxycarboxyl)-N-[but-3-en-2(R)-yl]hydroxylamine

A stirred mixture of the product from step (b) (200.0g) and 5% w/w Pd/BaSO4 (8.8g) in pyridine (1000ml) was hydrogenated at atmospheric pressure until hydrogen uptake ceased. The mixture was filtered through Hyflo, the filtrate evaporated in vacuo and the remaining oil taken up in ether (1000ml), washed with 20% w/v aqueous citric acid and water, dried and evaporated in vacuo to give the desired product (192.0g).

(d) 1-Bromo-3-(4-fluorophenoxy)benzene

An aqueous solution of KOH (49.0g in 100ml water) was added to a stirred solution of 4-fluorophenol (84.0g, Aldrich) in methanol (250ml). When addition was complete, the mixture was evaporated in vacuo and the residual solid pulverised and taken up in 1-methyl-2-pyrrolidinone (300ml). 3-Fluorobromobenzene (131.3g, Aldrich) was added and the mixture refluxed for 24 hours, then cooled to room temperature, poured into water (1500ml) and extracted with ether. The combined extracts were washed with 2M aqueous NaOH, 2M aqueous HCl and water, dried and evaporated in vacuo. The remaining oil was distilled to give the desired product (103.0g) at bp 85-100 C/0.25 mm Hg.

(e) (E) -N-{3-[3-(4-Fluorophenoxy)phenyl]-1(R)-methylprop-2-en-1-yl} hydroxylamine

A mixture of the product from step (c) (100.7g), the product from step (d) (103.0g), tri(o-tolyl)phosphine (10.0g) and palladium(II) acetate (4.0g) in tri(n-butyl)amine (300ml) was stirred at 115°C for 3 hours. The mixture was cooled to about 90°C, evaporated in vacuo and the residue taken up in ether (1000ml), washed with 33% w/v aqueous citric acid and filtered through Hyflo. The filtrate was washed with 10% w/v aqueous citric acid and water, dried and evaporated in vacuo to give a viscous tan oil. The latter was taken up in methanol (1000ml) and c.HCl (100ml) and the mixture refluxed for 1 hour, then cooled to room temperature and evaporated in vacuo. The residue was taken up in water (1000ml) and 10M aqueous NaOH (125ml), extracted with ether and the combined extracts washed with water, dried and evaporated in vacuo. The remaining tan oil was chromatographed through silica gel using ether/40-60 pet. ether (9:1) followed by ether to give the desired product as a yellow oil (21.9g).

(f) (E)-N-{3-[3-(4-Fluorophenoxy)phenyl]-1(R)-methylprop-2-en-1-yl}- N-hydroxyurea

2M aqueous HCl (160ml) was added dropwise at 0°C over 1 hour to a stirred solution of the product from step (e) (21.9g) and potassium cyanate (19.7g) in THF (190ml). When addition was complete, the mixture was

stirred at 0°C for 1.5 hours, then saturated aqueous NaCl was added and the organic phase removed. The aqueous phase was extracted with ether and the organic phase and extracts combined, washed with 2M aqueous HCl, saturated aqueous $NaHCO_3$, water and saturated aqueous NaCl, dried and evaporated in vacuo to give a cream solid which was recrystallised from ethyl acetate/40-60 pet. ether (2:1) to give colourless plates (17.7g) of mp 133-135°C (dec.). 200MHz $^1H$ NMR and IR were consistent with the desired product. Analysis: C 64.10% (64.54%); H 5.47% (5.42%); N 8.78% (8.86%). $[\alpha]_D^{23}$ +40.81°C (c = 1.0, EtOH).

Synthetic Example 3

Preparation of (E)-N-{3-[3-(4-fluorophenoxy]phenyl)-1(S)-methylprop-2-en-1-yl}-N-hydroxyurea

(a) But-3-yn-2(R)-ol

But-3-yn-2(R)-ol was obtained by the resolution of (±)-but-3-yn-2-ol (Aldrich) according to the procedure described in J. Med. Chem. 31, 1558 (1988).

(b) N,O-Bis(t-butoxycarbonyl)-N-[but-3-yn-2(S)-yl]hydroxylamine

DEAD (81.8g) was added dropwise at -70°C over 1 hour to a stirred solution of but-3-yn-2(R)-ol from step (a) (25.0g), triphenylphosphine (117.0g) and N,O-bis(t-butoxycarbonyl)hydroxylamine (83.2g, Fluka) in toluene (760ml). When addition was complete, the mixture was allowed to warm to room temperature, filtered and the filtrate evaporated in vacuo. The remaining oil was flash chromatographed twice through silica gel using DCM and ether/40-60 pet. ether (1:4) respectively to give the desired product as a pale yellow oil (98.6g).

(c) N,O-Bis(t-butoxycarbonyl)-N-[but-3-en-2(S)-yl]hydroxylamine

A stirred mixture of the product from step (b) (98.6g) and 10% w/w $Pd/BaSO_4$ (2.2g) in pyridine (500ml) was hydrogenated at atmospheric pressure until hydrogen uptake ceased. The mixture was filtered through Hyflo, the filtrate evaporated in vacuo and the remaining oil taken up in ether (500ml), washed with 20% w/v aqueous citric acid and water, dried and evaporated in vacuo to give the desired product as a pale orange oil (90.0g).

(d) 1-Bromo-3-(4-fluorophenoxy)benzene

1-Bromo-3-(4-fluorophenoxy)benzene was prepared by the method described in step (d) of Synthetic Example 2 supra.

(e) (E)-N-{3-[3-(4-Fluorophenoxy)phenyl]-1(S)-methylprop-2-en-1-yl}hydroxylamine

A mixture of the product from step (c) (57.4g), the product from step (d) (58.7g), tri(o-tolyl)phosphine (5.0g) and palladium(II) acetate (2.0g) in tri(n-butyl)amine (150ml) was stirred at 110-120°C for 3.5 hours. The mixture was cooled to room temperature, evaporated in vacuo and the residue taken up in ether (500ml) and filtered through Hyflo. The filtrate was washed with 10% w/v aqueous citric acid and water, dried and evaporated in vacuo to give a tan oil. The latter was taken up in methanol (500ml) and c.HCl (50ml) and the mixture refluxed for 1 hour, then cooled to room temperature and evaporated in vacuo. The residue was taken up in water (500ml) and 10M aqueous NaOH (60ml), extracted with ether and the combined extracts washed to neutrality with water, dried and evaporated in vacuo. The residue was flash chromatographed through silica gel using ether/40-60 pet. ether (1:1 (discarded) followed by 9:1) to give the desired product as a viscous pale yellow oil (32.5g).

(f) (E)-N-{3-[3-(4-fluorophenoxy)phenyl]-1(S)-methylprop-2-en-1-yl}-N-hydroxyurea

2M aqueous HCl (71.4ml) was added dropwise at 0°C over 45 minutes to a stirred solution of the product from step (e) (32.5g) and potassium cyanate (29.3g) in THF (282ml). When addition was complete, the mixture was stirred at 0°C for 45 minutes, then saturated aqueous NaCl was added and the organic phase removed. The aqueous phase was extracted with ether and the organic phase and extract combined, washed with 2M aqueous HCl and water, dried and evaporated in vacuo to give a sticky solid which was recrystallised from ethyl acetate to give colourless plates (25.8g) of mp 131-132.5°C (dec.). 200MHz $^1H$ NMR and IR were consistent

with the desired product. Analysis: C 64.37% (64.54%); H 5.42% (5.42%); N 8.79% (8.86%). $[\alpha]_D^{23}$ -41.61°C (c = 1.0, EtOH).

Synthetic Example 4

Preparation of (E)-N-{3-[3-(4-chlorophenoxy)phenyl]-1(R,S)-methylprop-2-en-1-yl}-N-hydroxyurea

(a) (E)-1-[3-(4-Chlorophenoxy)phenyl]buten-3-one

10N aqueous NaOH (2ml) was added to a solution of 3-(4-chlorophenoxy)-benzaldehyde (23.3g, Aldrich) in acetone (150ml) and stirred vigorously. After a few minutes' stirring, the temperature rose to 32°C. The mixture was left for 5 minutes and then poured into 2M aqueous HCl (600ml) and extracted with ether/pet. ether (1:1, 400ml). The extract was washed with water and saturated aqueous NaCl, dried over $MgSO_4$, and stipped to give the aldol product. The latter was taken up in toluene (300ml), p-toluenesulphonic acid (1g) was added, and the mixture heated for 1 hour. The mixture was then cooled, washed with saturated aqueous $NaHCO_3$ and saturated aqueous NaCl, dried over $MgSO_4$ and stripped. The residue was eluted through a silica gel column using methylene chloride/40-60 pet. ether (1:1, followed by 3:1) and the eluate stripped to give the desired product (19.0g).

(b) (E)-1-[3-(4-Chlorophenoxy)phenyl]buten-3-one oxime

The product from step (a) (19.0g) and hydroxylamine hydrochloride (6.9g) were taken up in methanol (100ml), pyridine (20ml) was added and the mixture stirred for 1 hour. The mixture was then stipped, redissolved in ether (200ml), washed with 2M aqueous HCl and saturated aqueous NaCl, dried over $MgSO_4$, and stripped to give the desired product (20.3g).

(c) (E)-N-{1-Methyl-3-[3(4-chlorophenoxy)phenyl]prop-2-enyl}hydroxylamine

The product from step (b) (20g) was taken up in methanol (100ml) and oxalic acid (31.3g) added. The mixture was cooled in an ice-bath and sodium cyanoborohydride (22g) added in portions under $N_2$ over 3 hours. The mixture was stirred for a further 1.5 hours, then more sodium cyanoborohydride (2g) was added and stirring continued for a further 1/2 hour. The mixture was then stripped and the residue partitioned between ethyl acetate (300ml) and water (300ml). The organic layer was separated, washed with saturated aqueous $NaHCO_3$ and saturated aqueous NaCl, then stripped to give the crude hydroxylamine.

(d) (E)-N-{1-Methyl-3-[3-(4-chlorophenoxy)phenyl]prop-2-en-1-yl}-N-hydroxyurea

A portion of the product from step (c) (6.0g) was taken up in THF (60ml) and potassium cyanate (4.9g) added at 0°C. After 15 minutes, 5M aqueous HCl (20ml) was added dropwise and the mixture stirred for 10 minutes. Saturated aqueous NaCl (100ml) and ether (100ml) were then added and the organic layer was separated, washed with 2M aqueous HCl (100ml) and saturated aqueous NaCl (100ml), dried over $MgSO_4$, and stripped to give the desired product which was recystallised from ethyl acetate/pet.ether. Yield 4.4g, mp 132-135°C.

Synthetic Example 5

Preparation of (E)-N-{3-[3-(4-chlorophenoxy)phenyl]-1(S)-methylprop-2-en-1-yl}-N-hydroxyurea

(a) (E)-1-[3-(4-Chlorophenoxy)phenyl]but-1-en-3-one

A mixture of 3-(4-chlorophenoxy)benzaldehyde (50.0g, Aldrich), dimethyl acetylmethylphosphonate (35.7g, Lancaster) and anhydrous $K_2CO_3$ (59.4g) in THF (300ml) was stirred under $N_2$ at 50-55°C for 20 hours. The cooled mixture was filtered, the residue washed with THF, and the combined filtrates evaporated in vacuo to give an orange oil which was flash chromatographed through a silica column using ether/40-60 pet. ether (1:1 v/v) as eluant to give the desired product as a viscous pale yellow oil (52.8g).

(b) (E)-1(R)-Methyl-3-[3-(4-chlorophenoxy)phenyl]prop-2-en-1-ol

$BH_3$.THF (1.0M, 96ml) and a solution of the product from step (a) (52.7g) in THF (total volume 96ml) were simultaneously added under $N_2$ to a stirred solution of (S)-3,3-diphenylpyrrolo-[1,2-c][1,3,2]oxazaborolidine. in THF (0.3H, 33ml, prepared by the method described in JOC 53, 2861 (1988)). After additions were complete, the mixture was stirred at room temperature for 15 minutes, then poured into ice/2M aqueous HCl (300g/300ml), stirred for 30 minutes, and extracted with ether (3 x 300ml). The combined extracts were washed with 2M aqueous HCl (200ml), saturated aqueous $NaHCO_3$ (200ml) and water (200ml), dried over $Na_2SO_4$ and evaporated in vacuo to give the desired product as a viscous yellow oil (55.3g) which partially crystallised from ether/40-60 pet. ether (1:1 v/v). The mother liquor was decanted off and flash chromatographed through a silica column to give the desired product as a pale yellow oil (25.7g), $[\alpha]_D^{23.5}$ +8.7° (c = 3.0, EtOH). 200MHz $^1H$ NMR and IR consistent with proposed structure.

(c) (E)-N,O-Bis(t-butoxycarbonyl)-N-{1(S)-methyl-3-[3-(4-chlorophenoxy)phenyl]prop-2-en-1-yl}hydroxylamine

A solution of DEAD (24.5g) in toluene (50ml) was added to a stirred mixture of the product from step (b) (25.7g), N,O-bis(t-butoxycarbonyl)hydroxylamine (22.9g, Fluka) and triphenylphosphine (36.8g) in toluene (375ml) at -65 to -70°C. After addition was complete, the mixture was stirred for 4 hours at -65 to -70°C, then further triphenylphosphine (5.0g) was added followed by further DEAD (3.3g) and the mixture stirred for another hour at -65 to -70°C. The warmed mixture was evaporated in vacuo to give a brown oil which was successively flash chromatographed through two silica columns using DCM and DCM/40-60 pet.ether/ether (3:6:0.25 v/v/v) respectively as eluants to give the desired product as a viscous yellow oil (20.3g).

(d) (E)-N-{1(S)-Methyl-3-[3-(4-chlorophenoxy)phenyl]prop-2-en-1-yl}hydroxylamine

Trifluoroacetic acid (28ml) was added under $N_2$ at 0°C to a stirred solution of the product from step (c) (20.3g) in DCM (112ml). After addition was complete, the mixture was stirred at room temperature for 3 hours, then ether (100ml) and water (100ml) were added. 10N aqueous NaOH (32ml) was added at 0°C to the vigorously stirred mixture. The mixture was basified with excess saturated aqueous $NaHCO_3$, extracted with ether (200ml), and the extract washed with saturated aqueous $NaHCO_3$ (100ml) and water (2 x 100ml), dried over $Na_2SO_4$ and evaporated in vacuo to give a viscous yellow oil which was flash chromatographed through a silica column using ether/pet.ether (4:1) to give the desired product, mp 83-84°C, $[\alpha]_D^{25}$ -31.1° (c = 1.2, $CHCl_3$).

(e)(E)-N-{1(S)-Methyl-3-[3-(4-chlorophenoxy)phenyl]prop-2-en-1-yl}-N-hydroxyurea

A portion of the product from step (d) (0.29g) was taken up in THF (10ml) and potassium cyanate (0.25g) added at 0°C. After 15 minutes, 2M aqueous HCl (2ml) was added dropwise and the mixture stirred for 2 hours. Saturated aqueous NaCl (50ml) and ether (50ml) were then added and the organic layer was separated, washed with saturated aqueous NaCl (5Oml), dried over $MgSO_4$, and stripped to give the desired product which was recrystalised from ethyl acetate/pet. ether. Yield 0.26g, mp 119-120°C, $[\alpha]_D^{23}$ -40.0° (c = 1.0, EtOH).

Synthetic Example 6

Preparation of (E)-N-{3-[3-(4-fluorophenoxy)phenyl]-1(R,S)-methylprop-2-en-1-yl}-N-hydroxyurea, sodium salt

The compound of Synthetic Example 1 (1.0g) was dissolved in a mixture of aqueous NaOH (0.11g in 25ml water) and methanol (30ml). The methanol was evaporated in vacuo and the remaining solution freeze-dried to give the desired product as a cream solid.

200MHz $^1H$ NMR (DMSO-$d_6$, ppm): 1.10-1.21 (3H, d, $-CH_3$), 4.75-4.92 (1H, m, >CH-), 5.27-6.14 (2H, bs, $-NH_2$), 6.22-6.51 (2H, s, -HC=CH-) and 6.69-7.37 (9H, m, aromatic); no -OH signal.

PHARMACEUTICAL FORMULATION EXAMPLES

The "active ingredient" in the following formulations is any compound of the invention (as hereinbefore defined, for example, any of the compounds of Synthetic Examples 1 to 5).

Example A: Tablet

| | Per tablet |
|---|---|
| Active Ingredient | 5.0 mg |
| Lactose | 82.0 mg |
| Starch | 10.0 mg |
| Povidone | 2.0 mg |
| Magnesium Stearate | 1.0 mg |

Mix together the active ingredient, lactose and starch. Granulate the powders using a solution of povidone in purified water. Dry the granules, add the magnesium stearate and compress to produce tablets (100mg per tablet).

Example B: Ointment

| | | |
|---|---|---|
| Active Ingredient | | 1.0 g |
| White Soft Paraffin | to | 100.0 g |

Disperse the active ingredient in a small volume of the vehicle. Gradually incorporate this into the bulk to produce a smooth, homogeneous product. Fill into collapsible metal tubes.

Example C: Cream for topical use

| | | |
|---|---|---|
| Active Ingredient | | 1.0 g |
| Polawax GP 200 | | 20.0 g |
| Lanolin Anhydrous | | 2.0 g |
| White Beeswax | | 2.5 g |
| Methyl hydroxybenzoate | | 0.1 g |
| Distilled Water | to | 100.0 g |

Heat the Polawax, beeswax and lanolin together at 60°C. Add a solution of methyl hydroxybenzoate. Homogenise using high speed stirring. Allow the temperature to fall to 50°C. Add and disperse the active ingredient. Allow to cool with slow speed stirring.

Example D: Lotion for topical use

| | | |
|---|---|---|
| Active Ingredient | | 1.0g |
| Sorbitan Monolaurate | | 0.6 g |
| Polysorbate 20 | | 0.6 g |
| Cetostearyl Alcohol | | 1.2 g |
| Glycerin | | 6.0 g |
| Methyl Hydroxybenzoate | | 0.2g |
| Purified Water B.P. | to | 100 ml |

The methyl hydroxybenzoate and glycerin were dissolved in 70ml of the water at 75°C. The sorbitan monolaurate, Polysorbate 20 and cetostearyl alcohol were melted together at 75°C and added to the aqueous solution. The resulting emulsion was homogenised, allowed to cool with continuous stirring and the active ingredient added as a suspension in the remaining water. The whole was stirred until homogeneous.

Example E: Eye drops

| | | |
|---|---|---|
| Active Ingredient | | 0.5 g |
| Methyl Hydroxybenzoate | | 0.01 g |
| Propyl Hydroxybenzoate | | 0.04 g |
| Purified Water B.P. | to | 100 ml |

The methyl and propyl hydroxybenzoates were dissolved in 70ml of purified water at 75°C and the resulting solution allowed to cool. The active ingredient was then added and the solution made up to 100ml with purified water. The solution was sterilised by filtration through a membrane filter of 0.22μm pore size and packed aseptically into suitable sterile containers.

Example F: Injectable solution

| | | |
|---|---|---|
| Active Ingredient | | 10.0 mg |
| Water for Injections B.P. | to | 1.0 g |

The active ingredient was dissolved in half of the Water for Injections and then made up to volume and sterilised by filtration. The resulting solution was distributed into ampoules under aseptic conditions.

Example G: Powder capsules for inhalation

| | |
|---|---|
| Active Ingredient (0.5-7.0μm powder) | 4 mg |
| Lactose (30-90μm powder) | 46.0 mg |

The powders were mixed until homogeneous and filled into suitably sized hard gelatin capsules (50mg per capsule).

Example H: Inhalation aerosol

| | | |
|---|---|---|
| Active Ingredient (0.5-7.0μm powder) | | 200 mg |
| Sorbitan Trioleate | | 100 mg |
| Saccharin Sodium (0.5-7.0μm powder) | | 5 mg |
| Methanol | | 2 mg |
| Trichlorofluoromethane | | 4.2 g |
| Dichlorodifluoromethane | to | 10.0 ml |

The sorbitan trioleate and menthol were dissolved in the trichlorofluoromethane. The saccharin sodium and active ingredient were dispersed in the mixture which was then transferred to a suitable aerosol canister and the dichlorofluoromethane injected through the valve system. This composition provides 2mg of active ingredient in each 100μl dose.

BIOLOGICAL DATA

Ex vivo inhibition of 5-lipoxygenase

Preliminary studies in rats and rabbits indicated that the racemate of Synthetic Example 1 and the corresponding enantiomers of Synthetic Examples 2 and 3 effectively inhibited the stimulated synthesis of leukotriene $B_4$ ($LTB_4$), a compound formed from arachidonic acid via the 5-lipoxygenase pathway.

After administration of the test compound, the concentration of $LTB_4$ in the plasma was measured periodically and expressed as a percentage of the mean control value. The procedure used is described in Br. J. Pharmacol. 94, 528 (1988) and may be summarised as follows.

Within 2 minutes of collection, duplicate samples (0.5ml) of blood were stimulated with the calcium ionophore A23187 (final concentration 15μg/ml) for 30 minutes at 37°C. When incubation was complete, the samples were centrifuged (12,000G for 2 minutes) and the cell-free plasma removed. The plasma concentration of $LTB_4$ was then determined by specific radioimmunoassay.

The compounds of Synthetic Examples 1 to 3 all inhibited the stimulated ex vivo synthesis of $LTB_4$ for several hours after their administration. The compound of Synthetic Example 1, for example, when administered orally to rabbits at a dose of 2mg/kg inhibited $LTB_4$ synthesis by more than 50% for a period of greater than 16 hours. The same compound when administered orally to rats at a dose of 10mg/kg inhibited $LTB_4$ synthesis by more than 50% for longer than 10 hours. The enantiomers of Synthetic Examples 2 and 3 produced similar

results.

In vitro inhibition of 5-lipoxygenase

Blood from normal aspirin-free volunteers was centrifuged to separate leukocytes from red cells and platelets. A solution of the test compound in DMSO (10μl, final concentration in the range 0.01 - 100μM) was added to cell homogenate (470μl) and the mixture incubated at 37°C for 5 minutes. Solutions of arachidonic acid in methanol (250μM, 10μl, final concentration 5μM) and calcium chloride in water (100mM, 10μl, final concentration 2mM) were then added simultaneously and the mixture incubated at 37°C for a further 5 minutes. The reaction was stopped by boiling. Radioimmunoassay was conducted for leukotriene $B_4$.

The experiment was repeated using ionophore-stimulated whole human blood.

For the compounds of Synthetic Examples 1 to 3, the results were as follows:

| | $IC_{50}$ (μM) | |
|---|---|---|
| Synthetic Example | Homogenate | Whole blood |
| 1 | 0.20 | 0.15 |
| 2 | 0.20 | 0.08 |
| 3 | 0.20 | 0.08 |

Anti-oxidant activity

As a measure of anti-oxidant activity, the peroxyl radical scavenging abilities of the compounds of the invention were determined by testing each compound's ability to inhibit the peroxidation of linoleic acid using the method described in Biochem. Pharm. 38, 1465 (1989).

The compound of Synthetic Example 1 had an apparent rate constant ($k_{AH}$) for scavenging peroxyl radicals of 0.408.

Combination effects with an NSAID

Male New Zealand White rabbits (2.5 - 3kg) were immunised with intradermal injections of 4mg of ovalbumin in 1ml of Freund's complete adjuvant. Animals were reimmunised 14 days later in the same way. Five days after the second immunisation, arthritis was induced in the right knee joint by injecting 1ml of a sterile solution of ovalbumin (5mg/ml) into the joint cavity.

Joint diameters were measured (on a randomised blind basis) with calipers at frequent intervals after antigen challenge. Animals were killed after 14 days and the joint space was washed with 1ml of saline and the resultant fluid was taken for total and differential leukocyte counts.

Sensitised animals were randomly allocated to groups of 5-8. The compound of Synthetic Example 1, (E)-N-{1-Methyl-3-[3-(4-fluorophenoxy)phenyl]prop-2-en-1-yl}-N-hydroxyurea (compound A), was administered in suspension following 18h ball-milling in 0.25% Celacol as vehicle. The non-steroidal anti-inflammatory indomethacin was dissolved in a small volume of 1M Tris buffer pH8 and then made up to volume with 0.25% Celacol. Control animals received 0.25% Celacol. Each animal received an oral dose (approximately 5ml) of vehicle or drug 3 times every 24h. Doses were administered at 8.0am, 4.0pm and 12 midnight for 16-17 days commencing 1h before antigen challenge on day 0. All doses were given orally by stomach tube. The groups were as follows:
1-7: Controls (5ml 0.25% Celacol x3/24h)
8-14: 1mg/kg indomethacin dissolved in 0.25% Celacol x3/24h
15-21: 2mg/kg compound A suspended in 0.25% Celacol x3/24h
22-28: 2mg/kg compound A suspended in 0.25% Celacol plus 1mg/kg indomethacin dissolved in 0.25% Celacol

Joint swelling

In control animals, antigen challenge caused an increase in joint diameter of 6-7mm which peaked 2 days after challenge. Joint swelling was sustained in the control group for the period of the experiment (14 days)

and by the time the animals were killed, the diameters of the arthritic joints were approximately 5mm greater than the contralateral joints. None of the drug treatments reduced joint swelling for the first 2 days after challenge, but from day 3 onwards, compound A reduced swelling by up to 26% and indomethacin by up to 53% compared to the vehicle-treated controls. The greatest and most consistent reductions in swelling (up to 72%) were seen in the group receiving a combination of compound A and indomethacin. Results are shown in Table 1.

TABLE 1

JOINT SWELLING (MM)

| Duration of arthritis | Compound A | Indomethacin | A + Indo. | Control |
|---|---|---|---|---|
| 1 | 4.6 | 4.3 | 3.9 | 5.6 |
| 2 | 5.2 | 4.5 | 4.2 | 6.0 |
| 3 | 4.6 | 3.5 | 3.1 | 5.7 |
| 8 | 3.5 | 2.6 | 1.6 | 4.3 |
| 14 | 3.2 | 2.0 | 1.2 | 4.3 |

Synovial fluid studies

Synovial lining tissue was removed from the joint, fixed, processed, cut and stained for microscopic examination. Histological scores were derived for the total mass of inflamed synovial lining, lymphocyte infiltration and polymorph content. The slides were read on a randomised blind basis.

Synovial fluids from the arthritic joints of control animals contained approximately $10^6$ leukocytes of which about 80% were polymorphs. Compound A or indomethacin alone both slightly reduced the mean leukocyte infiltrate in the synovial fluid, but the greatest reduction resulted from compound A with indomethacin. Results are shown in Table 2.

TABLE 2

LEUKOCYTES (CELL NUMBER x $10^6$)

| Compound A | Indomethacin | A + Indo. | Control |
|---|---|---|---|
| 6.1 | 7.3 | 0.9 | 7.9 |

Cartilage proteoglycan assay

Articular cartilage was dissected from the ends of the femurs and digested with papain prior to measurement of the concentration of sulphated glycosaminoglycans (GAGs). The proteoglycan content was expressed as µg GAG/mg wet weight of cartilage and compared with values obtained with cartilage from the contralateral control joint.

Articular cartilage dissected from the arthritic joints of vehicle-treated animals 14 days after antigen challenge contained 43% less proteoglycan than cartilage from the contralateral joints of the same animals. Compound A or indomethacin alone both reduced proteoglycan loss from the cartilage, but the greatest reduction was in the group receiving both compound A and indomethacin. Results are shown in Table 3.

TABLE 3

| PROTEOGLYCAN LOSS (%) | | | |
|---|---|---|---|
| Compound A | Indomethacin | A + Indo. | Control |
| 34 | 38 | 26 | 43 |

Histological evaluation of synovial lining

Both the lymphocyte and total cell count in the synovial lining of the group receiving both compound A and indomethacin were significantly reduced. Results are shown in Table 4.

TABLE 4

| | Compound A | Indomethacin | A + Indo. | Control |
|---|---|---|---|---|
| Lymphocytes | 59 | 51 | 36 | 68 |
| Total cells | 367 | 365 | 255 | 435 |

The foregoing data demonstrate the cytoprotective effect of compound A in arthritis, especially in the presence of the non-steroidal anti-inflammatory drug indomethacin.

Toxicity data

The compound of Synthetic Example 1 was administered to mice per orally at doses of up to 300mg/kg and intraperitoneally at doses of up to 100mg/kg. No deaths or serious deleterious effects were observed.

## Claims

### Claims for the following Contracting States : AT, BE, CH, DE, DK, FR, GB, IT, LI, LU, NL, SE

1. A compound of formula (I)

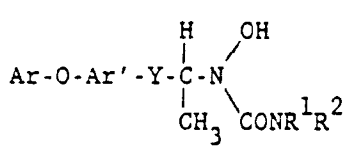

(I)

wherein
Ar is 4-halophenyl;
Ar' is 1,3- or 1,4-phenylene;
Y is (E)-CH=CH-; and
$R^1$ and $R^2$ are independently selected from hydrogen and $C_{1-4}$, alkyl;
and salts and physiologically functional derivatives thereof.

2. A compound as claimed in claim 1, wherein

Ar is 4-fluorophenyl; and/or
Ar' is 1,3-phenylene; and/or
$R^1$ and $R^2$ are both hydrogen;
and salts and physiologically functional derivatives thereof.

3. (E)-N-(1-Methyl-3-[3-(4-fluorophenoxy)phenyl]prop-2-en-1-yl)-N-hydroxyurea in either its (R) or (S) enantiomeric form or a mixture thereof in any proportions, and physiologically acceptable salts and physiologically functional derivatives thereof.

4. The compound as claimed in claim 3, which compound is in its (R) enantiomeric form and is substantially free of the (S)-isomer.

5. The compound as claimed in claim 3, which compound is in its (S) enantiomeric form and is substantially free of the (R)-isomer.

6. The compound as claimed in claim 3, which compound is in its racemic form.

7. A pharmaceutical formulation comprising a compound as claimed in claim 1, a pharmaceutically acceptable carrier or excipient and, optionally, one or more other pharmacologically active ingredients.

8. A formulation as claimed in claim 7, wherein the hydroxyurea compound is as claimed in claim 2.

9. A formulation as claimed in claim 8, wherein the hydroxyurea compound is (E)-N-{1-methyl-3-[3-(4-fluorophenoxy)phenyl]prop-2-en-1-yl}-N-hydroxyurea in either its (R) or (S) enantiomeric form or a mixture thereof in any proportions.

10. A formulation as claimed in claim 9, wherein the hydroxyurea compound is in its (R) enantiomeric form and is substantially free of the (S)-isomer.

11. A formulation as claimed in claim 9, wherein the hydroxyurea compound is in its (S) enantiomeric form and is substantially free of the (R)-isomer.

12. A formulation as claimed in claim 9, wherein the hydroxyurea compound is in its racemic form.

13. A formulation as claimed in any of claims 7 to 12 which includes one or more other pharmacologically active ingredients selected from antibiotic, anti-fungal, anti-viral, anti-histamine and non-steroidal anti-inflammatory drugs.

14. A formulation as claimed in claim 13 which includes one or more non-steroidal anti-inflammatory drugs.

15. A formulation as claimed in any of claims 7 to 14 which is in a form suitable for oral, topical, pulmonary, or intra-articular administration.

16. A process for the preparation of a compound of formula (I) as claimed in claim 1 which comprises reacting a compound of formula (II)

$R^3NHOH$ (II)

wherein $R^3$ is a group of formula

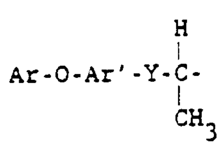

as defined in claim 1, with a suitable agent or agents and under such reaction conditions as to effect conversion of the N-hydrogen to an N-$CONR^1R^2$ group;
and optional conversion of the compound of formula (I) so formed to a corresponding base salt or physiologically functional derivative.

17. A compound as claimed in any of claims 1 to 6 for use in therapy.

**18.** Use of a compound as claimed in any of claims 1 to 6 in the manufacture of a medicament for the prophylaxis or treatment of a clinical condition for which a 5-lipoxygenase inhibitor is indicated.

**19.** Use of a compound as claimed in any of claims 1 to 6 in the manufacture of a medicament for the prophylaxis or treatment of a clinical condition(s) which condition(s) is/are one or more selected from spasmogenic conditions, allergic conditions, tumour formation, conditions involving blood platelet aggregation, and inflammatory conditions.

**20.** Use as claimed in claim 19 wherein the clinical condition is intrinsic (spasmogenic) or extrinsic (allergic) asthma or irritable (spasmogenic) bowel syndrome.

**21.** Use of a compound as claimed in any of claims 1 to 6 in the manufacture of a medicament for the prophylaxis or treatment of a clinical condition for which an anti-oxidant is indicated.

**22.** Use of a compound as claimed in any of claims 1 to 6 in the manufacture of a medicament for the prophylaxis or treatment of atherosclerosis or arthritis.

**Claims for the following Contracting States : ES, GR**

**1.** A process for the preparation of a compound of formula (I)

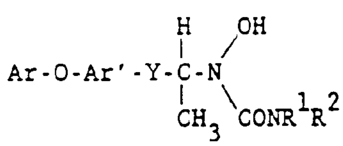

(I)

wherein
Ar is 4-halophenyl;
Ar' is 1,3- or 1,4-phenylene;
Y is (E)-CH=CH-; and
$R^1$ and $R^2$ are independently selected from hydrogen and $C_{1-4}$ alkyl;
which process comprises reacting a compound of formula (II)

$R^3NHOH$ (II)

wherein $R^3$ is a group of formula

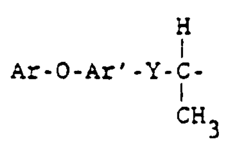

as defined above, with a suitable agent or agents and under such reaction conditions as to effect conversion of the N-hydrogen to an N-$CONR^1R^2$ group;
and optional conversion of the compound of formula (I) so formed to a corresponding base salt or physiologically functional derivative.

**2.** A process as claimed in claim 1 for the preparation of a compound of formula (I) as shown in claim 1, wherein
Ar is 4-fluorophenyl; and/or
Ar' is 1,3-phenylene; and/or
$R^1$ and $R^2$ are both hydrogen.

**3.** A process as claimed in claim 2 for the preparation of a compound as defined in claim 1, which compound is (E)-N-{1-methyl-3-[3-(4-fluorophenoxy)phenyl]prop-2-en-1-yl}-N-hydroxyurea in either its (R) or (S) enantiomeric form or a mixture thereof in any proportions.

**4.** A process as claimed in any of claims 1 to 3, wherein the compound of formula (II) is optically active and

the compound of formula (I) is obtained in its (R) enantiomeric form and is substantially free of the (S)-isomer.

5. A process as claimed in any of claims 1 to 3, wherein the compound of formula (II) is optically active and the compound of formula (I) is obtained in its (S) enantiomeric form and is substantially free of the (R)-isomer.

6. A process as claimed in any of claims 1 to 3, wherein the compound of formula (II) is optically inactive and the compound of formula (I) is obtained in its racemic form.

7. A method of preparing a pharmaceutical formulation which comprises
   (a) preparing a compound as defined in claim 1 by a process as claimed in any of claims 1 to 6; and
   (b) admixing or dissolving/suspending the product from step (a) and, optionally, one or more other pharmacologically active ingredients with/in a pharmaceutically acceptable carrier or excipient.

8. A method as claimed in claim 7 wherein step (b) comprises admixing or dissolving/suspending one or more other pharmacologically active ingredients selected from antibiotic, anti-fungal, anti-viral, anti-histamine and non-steroidal anti-inflammatory drugs with/in the carrier or excipient.

9. A method as claimed in claim 8 wherein step (b) comprises admixing or dissolving/suspending one or more non-steroidal anti-inflammatory drugs with/in the carrier or excipient.

10. A method as claimed in any of claims 7 to 9 which comprises an additional step (c) wherein the product from step (b) is rendered suitable for oral, topical, pulmonary, or intra-articular administration.

## Patentansprüche

### Patentansprüche für folgende Vertragsstaaten : AT, BE, CH, DE, DK, FR, GB, IT, LI, LU, NL, SE

1. Eine Verbindung der Formel (I)

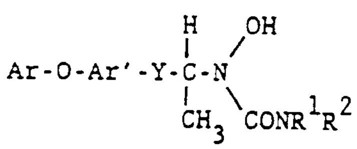

(I)

   worin
   Ar ist 4-Halogenphenyl;
   Ar' ist 1,3- oder 1,4-Phenylen;
   Y ist (E)-CH=CH- ; und
   $R^1$ und $R^2$ sind unabhängig voneinander ausgewählt aus Wasserstoff und $C_{1-4}$ Alkyl;
   und Salze und physiologisch wirksame Derivate davon.

2. Verbindung nach Anspruch 1, worin
   Ar ist 4-Fluorphenyl; und/oder
   Ar' ist 1,3-Phenylen; und/oder
   $R^1$ und $R^2$ sind beide Wasserstoff;
   und Salze und physiologisch wirksame Derivate davon.

3. Verfahren nach Anspruch 2 zur Herstellung einer Verbindung wie in Anspruch 1 definiert, wobei die Verbindung (E)-N-(1-Methyl)-3-[3-(4-fluorphenoxy)phenyl]prop-2-en-1-yl)-N-hydroxyharnstoff, entweder in seiner (R)- oder (S)-enantiomeren Form, oder als Mischung davon in beliebigen Anteilen, und physiologisch annehmbare Salze und physiologisch wirksame Derivate davon.

4. Verbindung nach Anspruch 3, worin die Verbindung in ihrer (R)-enantiomeren Form vorliegt und im wesentlichen frei vom (S)-Isomer ist.

5. Verbindung nach Anspruch 3, worin die Verbindung in ihrer (S)-enantiomeren Form vorliegt und im wesentlichen frei vom (R)-Isomer ist.

6. Verbindung nach Anspruch 3, worin die Verbindung als Razemat vorliegt.

7. Pharmazeutische Formulierung, enthaltend eine Verbindung gemäß Anspruch 1, einen pharmazeutisch annehmbaren Träger oder Vehikel und wahlweise einen oder mehrere andere pharmakologisch aktive Bestandteile.

8. Formulierung nach Anspruch 7, worin der Hydroxyharnstoff eine Verbindung nach Anspruch 2 ist.

9. Formulierung nach Anspruch 8, worin die Hydroxyharnstoffverbindung (E)-N-(1-Methyl-3-[3-(4-fluorphenoxy)phenyl]-prop-2-en-1-yl)-N-hydroxyharnstoff entweder in ihrer (R)- oder (S)- enantiomeren Form oder eine Mischung davon in beliebigen Anteilen ist.

10. Formulierung nach Anspruch 9, worin die Hydroxyharnstoffverbindung in ihrer (R)-enantiomeren Form vorliegt und im wesentlichen frei vom (S)-Isomer ist.

11. Formulierung nach Anspruch 9, worin die Hydroxyharnstoffverbindung in ihrer (S)-enantiomeren Form vorliegt und im wesentlichen frei vom (R)-Isomer ist.

12. Formulierung nach Anspruch 9, worin die Hydroxyharnstoffverbindung als Razemat vorliegt.

13. Formulierung nach einem der Ansprüche 1 bis 12, welche einen oder mehrere andere pharmakologisch wirksame Bestandteile, ausgewählt aus Antibiotika, Antifungiziden, antiviralen Mitteln, Antihistaminika und nicht-steroiden, entzündungshemmenden Arzneimitteln umfaßt.

14. Formulierung nach Anspruch 13, welche ein oder mehrere nicht-steroide entzündungshemmende Arzneimittel umfaßt.

15. Formulierung nach einem der Ansprüche 7 bis 14, welche in geeigneter Form zur oralen, topischen, pulmonären oder intraartikulären Verabreichung vorliegt.

16. Verfahren zur Herstellung einer Verbindung der Formel (I) gemäß Anspruch 1, welches umfaßt Umsetzen einer Verbindung der Formel (II)

$$R^3NHOH \qquad (II)$$

worin $R^3$ eine Gruppe der Formel

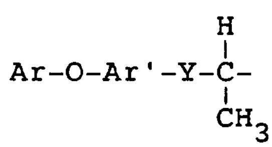

wie in Anspruch 1 definiert darstellt, mit einem geeigneten Mittel oder Mitteln unter solchen Reaktionsbedingungen, daß die Umwandlung des N-Wasserstoffs in eine N-$CONR^1R^2$-Gruppe erfolgt; und wahlweise Umwandlung der so gebildeten Verbindung der Formel (I) in ein entsprechendes basisches Salz oder physiologisch wirksames Derivat.

17. Verbindung nach einem der Ansprüche 1 bis 6 zur Verwendung bei der Therapie.

18. Verwendung einer Verbindung, wie in einem der Ansprüche 1 bis 6 beansprucht, zur Herstellung eines Medikaments zur Prophylaxe oder Behandlung eines klinischen Zustandes, für den ein 5-Lipoxygenase-Inhibitor indiziert ist.

19. Verwendung einer Verbindung nach einem der Ansprüche 1 bis 6 bei der Herstellung eines Medikaments zur Prophylaxe oder Behandlung eines klinischen Zustands (von klinischen Zuständen), bei denen der Zustand (die Zustände) ausgewählt ist (sind) aus spasmogenen Zuständen, allergischen Zuständen, Tumorbildung, Zuständen, die mit Blutplättchenaggregation verbunden sind, und Entzündungszuständen.

20. Verwendung nach Anspruch 19, worin der klinische Zustand ein intrinsisches (spasmogenes) oder extrinsisches (allergisches) Asthma oder ein (spasmogenes) Reizsyndrom des Darms ist.

21. Verwendung einer Verbindung nach einem der Ansprüche 1 bis 6 bei der Herstellung eines Medikaments zur Prophylaxe oder Behandlung eines klinischen Zustandes, bei dem ein Antioxidationsmittel indiziert ist.

22. Verwendung einer Verbindung nach einem der Ansprüche 1 bis 6 bei der Herstellung eines Medikaments zur Prophylaxe oder Behandlung von Arteriosklerose oder Arthritis.

**Patentansprüche für folgende Vertragsstaaten : ES, GR**

1. Verfahren zur Herstellung einer Verbindung der Formel (I)

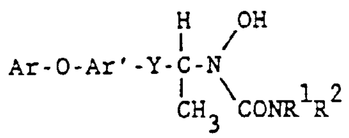

(I)

worin
Ar ist 4-Halogenphenyl;
Ar' ist 1,3- oder 1,4-Phenylen;
Y ist (E)-CH=CH-; und
$R^1$ und $R^2$ sind unabhängig voneinander ausgewählt aus Wasserstoff und $C_{1-4}$-Alkyl;
wobei das Verfahren umfaßt Umsetzen einer Verbindung der Formel (II)

$R^3NHOH$ (II)

worin $R^3$ eine Gruppe der Formel

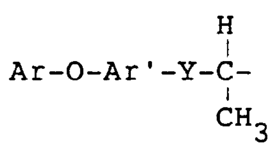

wie oben definiert darstellt, mit einem geeigneten Mittel oder Mitteln unter solchen Reaktionsbedingungen, um die Konversion des N-Wasserstoffs in eine $N-CONR^1R^2$-Gruppe zu bewirken, und wahlweises Umwandeln der so gebildeten Verbindung der Formel (I) in ein entsprechendes basisches Salz oder ein physiologisch wirksames Derivat.

2. Verfahren nach Anspruch 1 zur Herstellung einer Verbindung der Formel (I), wie in Anspruch 1 gezeigt, worin
Ar ist 4-Fluorphenyl; und/oder
Ar' ist 1,3-Phenylen; und/oder
$R^1$ und $R^2$ sind beide Wasserstoff.

3. Verfahren nach Anspruch 2 zur Herstellung einer Verbindung wie in Anspruch 1 definiert, wobei die Verbindung (E)-N-(1-Methyl-3-[3-(4-fluorphenoxy)phenyl]prop-2-en-1-yl)-N-hydroxy-harnstoff entweder in seiner (R)- oder (S)-enantiomeren Form oder eine Mischung davon in beliebigen Anteilen ist.

4. Verfahren nach einem der Ansprüche 1 bis 3, worin die Verbindung der Formel (II) optisch aktiv ist, und die Verbindung der Formel (I) als (R)-Enantiomer erhalten wird und im wesentlichen frei von (S)-Isomer ist.

5. Verfahren nach einem der Ansprüche 1 bis 3, worin die Verbindung der Formel (II) optisch aktiv ist, und die Verbindung der Formel (I) in ihrer (S)-enantiomeren Form erhalten wird und im wesentlichen frei vom (R)-Isomer ist.

6. Verfahren nach einem der Ansprüche 1 bis 3, worin die Verbindung der Formel (II) optisch inaktiv ist, und

die Verbindung der Formel (I) im wesentlichen als Razemat erhalten wird.

**7.** Verfahren zur Herstellung einer pharmazeutischen Formulierung, welche umfaßt
(a) Herstellung einer Verbindung, definiert in Anspruch 1 nach einem Verfahren, wie in einem der Ansprüche 1 bis 6 beansprucht, und
(b) Mischen oder Lösen/Suspendieren des Produkts aus Schritt (a) und wahlweise einem oder mehreren anderen pharmakologisch wirksamen Bestandteilen mit/in einem pharmazeutisch annehmbaren Träger oder Vehikel.

**8.** Verfahren nach Anspruch 7, worin Schritt (b) das Mischen oder Lösen/Suspendieren von einem oder mehreren anderen pharmakologisch wirksamen Bestandteilen, ausgewählt aus Antibiotika, Antifungiziden, antiviralen Mitteln, Antihistaminika und nicht-steroiden, entzündungshemmenden Arzneimitteln mit/in einem Träger oder Vehikel umfaßt.

**9.** Verfahren nach Anspruch 8, worin Schritt (b) das Vermischen oder Lösen/Suspendieren von einem oder mehreren nicht-steroiden, entzündungshemmenden Mitteln mit/in einem Träger oder Vehikel umfaßt.

**10.** Verfahren nach einem der Ansprüche 7 bis 9, welches den zusätzlichen Schritt (c) umfaßt, wobei das Produkt aus Schritt (b) für orale, topische, pulmonare oder intraartikuläre Verabreichung geeignet hergerichtet wird.

## Revendications

### Revendications pour les Etats contractants suivants : AT, BE, CH, DE, DK, FR, GB, IT, LI, LU, NL, SE

**1.** Composé de formule I :

```
                H   OH
                |  /
Ar-O-Ar'-Y-C-N                          (I)
                |  \
               CH3  CONR1R2
```

où
Ar est 4-halophényle;
Ar' est 1,3- ou 1,4-phénylène;
Y est (E)-CH=CH-, et
$R^1$ et $R^2$ sont choisis indépendamment parmi hydrogène et $C_{1-4}$-alcoyle;
et ses sels et dérivés physiologiquement fonctionnels.

**2.** Composé suivant la revendication 1, dans lequel :
Ar est 4-fluorophényle; et/ou
Ar' est 1,3-phénylène; et/ou
$R^1$ et $R^2$ sont tous deux hydrogène,
et ses sels et dérivés physiologiquement fonctionnels.

**3.** La (E)-N-{1-méthyl-3-[3-(4-fluorophénoxy)-phényl] prop-2-ène-1-yl}-N-hydroxyurée sous sa forme énantiomère (R) ou (S) ou un mélange de celles-ci en proportions quelconques et leurs sels physiologiquement acceptables et dérivés physiologiquement fonctionnels.

**4.** Composé suivant la revendication 3, lequel composé se trouve sous sa forme énantiomère (R) et est sensiblement exempt de l'isomère (S).

**5.** Composé suivant la revendication 3, lequel composé se trouve sous sa forme énantiomère (S) et est sensiblement exempt de l'isomère (R).

**6.** Composé suivant la revendication 3, lequel composé se trouve sous sa forme racémique.

**7.** Composition pharmaceutique comprenant un composé suivant la revendication 1, un porteur ou excipient pharmaceutiquement acceptable et facultativement un ou plusieurs autres constituants pharmacologiquement actifs.

**8.** Composition suivant la revendication 7, dans laquelle l'hydroxyurée est telle que revendiquée dans la revendication 2.

**9.** Composition suivant la revendication 8, dans laquelle l'hydroxyurée est la (E)-N-{1-méthyl-3-[3-(4-fluorophénoxy)phényl]prop-2-ène-1-yl}-N-hydroxyurée sous sa forme énantiomère (R) ou (S) ou un mélange de celles-ci en proportions quelconques.

**10.** Composition suivant la revendication 9, dans laquelle l'hydroxyurée se trouve sous sa forme énantiomère (R) et est sensiblement exempte de l'isomère (S).

**11.** Composition suivant la revendication 9, dans laquelle l'hydroxyurée se trouve sous sa forme énantiomère (S) et est sensiblement exempte de l'isomère (R).

**12.** Composition suivant la revendication 9, dans laquelle l'hydroxyurée se trouve sous sa forme racémique.

**13.** Composition suivant l'une quelconque des revendications 7 à 12, qui comprend un ou plusieurs constituants pharmacologiquement actifs choisis parmi les médicaments antibiotiques, antifongiques, antiviraux, antihistaminiques et anti-inflammatoires non stéroïdiens.

**14.** Compositions suivant la revendication 13, qui comprend un ou plusieurs médicaments anti-inflammatoires non stéroïdiens.

**15.** Composition suivant l'une quelconque des revendications 7 à 14, qui est présentée sous une forme se prêtant à l'administration par voie orale, topique, pulmonaire ou intra-articulaire.

**16.** Procédé pour préparer un composé de formule (I) suivant la revendication 1, qui comprend la réaction d'un composé de formule II :

$$R^3NHOH \qquad (II)$$

où $R^3$ est un radical de formule :

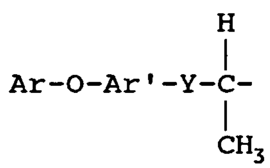

tel que défini à propos d'un composé de formule I, avec un ou des agents appropriés et dans des conditions de réaction propres à opérer la conversion du radical N-H en un radical $N\text{-}CONR^1R^2$;
et la conversion facultative du composé de formule (I) ainsi obtenu en un sel de base ou dérivé physiologiquement fonctionnel correspondant.

**17.** Composé suivant l'une quelconque des revendications 1 à 6, à utiliser en thérapeutique.

**18.** Utilisation d'un composé suivant l'une quelconque des revendications 1 à 6, dans la fabrication d'un médicament pour la prophylaxie ou le traitement d'un état clinique dans lequel un inhibiteur de la 5-lipoxygénase est indiqué.

**19.** Utilisation d'un composé suivant l'une quelconque des revendications 1 à 6, dans la fabrication d'un médicament pour la prophylaxie ou le traitement d'un ou plusieurs états cliniques qui sont choisis parmi les états spasmodiques, les états allergiques, la formation d'une tumeur, les états impliquant l'agrégation des plaquettes du sang et les états inflammatoires.

**20.** Utilisation suivant la revendication 19, dans laquelle l'état clinique est l'asthme intrinsèque (spasmogène) ou extrinsèque (allergique) ou le syndrome des intestins irritables (spasmogène).

**21.** Utilisation d'un composé suivant l'une quelconque des revendications 1 à 6, dans la fabrication d'un médicament pour la prophylaxie ou le traitement d'un état clinique pour lequel un antioxydant est indiqué.

**22.** Utilisation d'un composé suivant l'une quelconque des revendications 1 à 6, dans la fabrication d'un médicament pour la prophylaxie ou le traitement de l'athérosclérose ou de l'arthrite.

**Revendications pour les Etats contractants suivants : ES, GR**

**1.** Procédé pour préparer un composé de formule I :

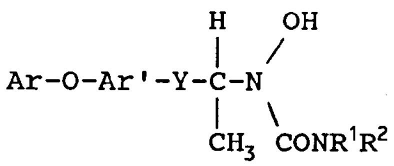

(I)

où
Ar est 4-halophényle;
Ar' est 1,3- ou 1,4-phénylène;
Y est (E)-CH=CH-, et
$R^1$ et $R^2$ sont choisis indépendamment parmi hydrogène et $C_{1-4}$-alcoyle;
lequel procédé comprend la réaction d'un composé de formule (II) :

$R^3NHOH$ (II)

où $R^3$ est un radical de formule :

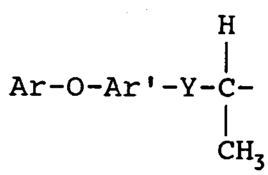

tel que défini ci-dessus, avec un ou des agents appropriés et dans des conditions de réaction propres à opérer la conversion du radical N-H en un radical N-$CONR^1R^2$;
et la conversion facultative du composé de formule (I) ainsi obtenu en un sel de base ou dérivé physiologiquement fonctionnel correspondant.

**2.** Procédé suivant la revendication 1, pour préparer un composé de formule (I) tel que défini dans la revendication 1, dans lequel :
Ar est 4-fluorophényle; et/ou
Ar' est 1,3-phénylène; et/ou
$R^1$ et $R^2$ sont tous deux hydrogène.

**3.** Procédé suivant la revendication 2 pour préparer un composé tel que défini dans la revendication 1, lequel composé est la (E)-N-{1-méthyl-3-[3-(4-fluorophénoxy)phényl]prop-2-ène-1-yl}-N-hydroxyurée sous sa forme énantiomère (R) ou (S) ou un mélange de celles-ci en proportions quelconques.

**4.** Procédé suivant l'une quelconque des revendications 1 à 3, dans lequel le composé de formule (II) est optiquement actif et le composé de formule (I) est obtenu sous sa forme énantiomère (R) et est sensiblement exempt de l'isomère (S).

**5.** Procédé suivant l'une quelconque des revendications 1 à 3, dans lequel le composé de formule (II) est optiquement actif et le composé de formule (I) est obtenu sous sa forme énantiomère (S) et est sensiblement exempt de l'isomère (R).

**6.** Procédé suivant l'une quelconque des revendications 1 à 3, dans lequel le composé de formule (II) est optiquement inactif et le composé de formule (I) est obtenu sous sa forme racémique.

**7.** Procédé pour préparer une composition pharmaceutique, qui comprend :

(a) la préparation d'un composé tel que défini dans la revendication 1 par un procédé suivant l'une quelconque des revendications 1 à 6, et

(b) le mélange ou la dissolution/mise en suspension du produit du stade (a) et, facultativement, d'un ou plusieurs autres constituants pharmacologiquement actifs avec/dans un porteur ou excipient pharmaceutiquement acceptable.

**8.** Procédé suivant la revendication 7, dans lequel le stade (b) comprend le mélange ou la dissolution/mise en suspension d'un ou plusieurs autres constituants pharmacologiquement actifs choisis parmi les médicaments antibiotiques, antifongiques, antiviraux, antihistaminiques et anti-inflammatoires non stéroïdiens avec/dans le porteur ou excipient.

**9.** Procédé suivant la revendication 8, dans lequel le stade (b) comprend le mélange ou la dissolution/mise en suspension d'un ou plusieurs médicament anti-inflammatoires non stéroïdiens avec/dans le porteur ou excipient.

**10.** Procédé suivant l'une quelconque des revendications 7 à 9, qui comprend un stade supplémentaire (c) dans lequel le produit du stade (b) est rendu propre à l'administration par voie orale, topique, pulmonaire ou intra-articulaire.